# EUROPEAN PATENT APPLICATION

(11) **EP 2 813 211 A1**
(43) Date of publication of application: **17.12.2014**
(21) Application number: 13746172.9
(22) Date of filing: 08.02.2013
(51) Int. Cl.: A61K 8/98, A61K 8/02, A61K 35/28, A61K 35/32, A61N 1/30, A61P 17/00, A61P 43/00, A61Q 19/08

(54) **COSMETIC PRODUCT OR SKIN REGENERATION PROMOTER COMPRISING NONHUMAN STEM CELL CULTURE SUPERNATANT AS STARTING MATERIAL, AND METHOD FOR ION INTRODUCTION FOR PROTEIN**

(30) Priority: 10.02.2012 JP 2012027852
(71) Applicant: Japanic Corporation, Tokyo 194-0003 (JP)
(72) Inventor: UEDA, Minoru, Nagoya-shi Aichi 461-0011 (JP); YAMASHITA, Yasuhiro, Machida-shi Tokyo 194-0003 (JP)
(74) Representative: Kuhnen & Wacker
(86) International application number: PCT/JP2013/053094
(87) International publication number: WO 2013/118877

(57) **Abstract**

As the percentage of senior citizens increases at the present time, the purpose of the present invention is to provide a cosmetic which can maintain the skin in a healthy state by preventing damage to the skin that accompanies aging, more specifically, discoloration and wrinkling, is very safe, does not pose ethical problems, and can be supplied in a sufficient amount. The present invention provides a cosmetic that comprises as the main component a powder of the supernatant produced by culturing the bone marrow or dental pulp stem cells of a nonhuman mammal. Moreover, provided is a method for ion introduction for protein by which the cosmetic is introduced by ion introduction.

## Description

### Technical Field

The present invention relates to a cosmetic including culture sup of a stem cell selected from the group consisting of non-human dental pulp stem cell, bone marrow stem cell, and adipose stem cell.

### Background Art

It is known that aged people have more blotches and wrinkles, which is caused by increasing amount of exposure to ultraviolet B (UVB) with advancing age. It is considered that UVB increases production amount of collagenase by human dermal fibroblast in dermis (HDF) so that it accelerates to collagen lysis in dermis and induces the deposition of degenerated elastic tissue, resulting the wrinkles and yellowing the skin.

Since ancient times, cosmetics have been produced by using plant derived ingredients as raw materials and used. In order to prevent to weaken the skin by aging, for example, such as wrinkle and fine lines formation and to improve moisture-retaining property, recently, the cosmetic combined with animal derived ingredients such as placenta, squalane, collagen and the like became commercially available widely.

Also, in order to achieve the same purposes, the cosmetics including culture sup of cells has been developed. It is proposed to culture smooth muscle cells of human lung, epidermal cells, and fibroblast cells, and homogenates thereof or culture supernatant to use them as components for the cosmetics (see JP 2005 - 336188 A, herein below, it is referred to as the prior art 1 ).

On the other hand, it is known a method to use stem cells from human bone marrow, dental pulp and the like, not but the culture sup, as a composition for treating neurological disease (WO 02/086108, herein below, it is referred to as the prior art 2).

Alternatively, it is proposed that the culture sup of human stem cells from exfoliated deciduous tooth (stem cells from exfoliated deciduous tooth; SHED) is used as the composition for treating lesion site (international publication WO 2011/118795, herein below, it is referred to as the prior art 3).

### Prior art documents

### Patent documents

Patent document 1: JP 2005-336188 A
Patent document 2: International publication WO 02/086108
Patent document 3: International publication WO 2011/118795

### Summary of the Invention

### Problem to be solved by the Invention

In the prior art 1, a technique for combining leukemia inhibitory factor (lymphoma inhibition factor: LIF), LIF analogs, or LIF mimic (herein below, they are collectively referred to as "LIFs") with the components for cosmetics is proposed. The prior art 1 is excellent from the view point that the high quality epidermis is formed by adding the culture sup containing LIF to cryopreserved epidermis in vitro.

However, it is not promised that of experimental results in vivo is reflected to that in vivo. Therefore, there is no proof for the cosmetic to have the same effect in vitro and in vivo.

Also, the prior art 2 is excellent from the view point that the stem cell having higher growth ability is used to utilize biologic factors produced by the stem cells.

However, since it uses the human-derived stem cell, there are problems such as: (a) number of the stem cell to be obtained is decreasing by aging; (b) safety for transplantation of the stem cell is not always secured, because gene mutations caused by the aging are accumulated; (c) the stem cell has low cellular proliferative potential, although it is the stem cell, because the number, the proliferative potential, and differential potential of the bone marrow cell (BMSC) become lower by the aging; (d) it is heavily invasive and dangerous to obtain the stem cell through bone marrow puncture. Also, use of human cells causes troubles that it is difficult to provide enough amounts of stem cell source, and also in ethical aspects.

The prior art 3 uses the culture sup of the human dental pulp stem cells. By this, it is excellent that it is safe that the use of the cell by themselves.

However, as the same as the prior art 2, it uses the human cells, the exfoliated tooth, even though they are referred to as medical waste. Therefore, it has the problem in the ethical aspect, and also has the difficulty to provide enough amounts of the stem cell resources.

On the other hand, it is thought that a pluripotent stem cell may differentiate into many tissues such as dermal tissue, bone tissue, muscle tissue and others. At present, since a ratio of aging population becomes high, there is strong need to maintain the dermis in healthy condition by preventing to dermal lesion caused by the aging, namely, to prevent to form spots and blotches, wrinkles and the like. Also, there is the need to solve the problem related to hair, such as thinning hair, fallen hair and the like, regardless of age.

Also, there is the strong need to the cosmetic having the effects to reduce or recover damages from UVB.

### Problems to be solved by the invention

Under such circumstances, the inventors of the present invention have continued to study to solve the above-mentioned problems. Then, they have completed the present invention.

The first aspect of the present invention is a cosmetic comprising any one of powdery culture sup of stem cell selected form the group consisting of a non-human mammal dental pulp stem cell, a non-human bone marrow stem cell and a non-human adipose stem cell as a main ingredient. Here, the non-human stem cell is preferably obtained from any one of tissue selected from the group consisting of a swine dental pulp of exfoliated deciduous teeth, a swine bone marrow, and a swine adipose. Also, the powdery culture sup is preferably prepared from a culture sup selected from the group consisting of the swine dental pulp of the exfoliated deciduous teeth, the swine bone marrow, and the swine adipose tissue by freeze-drying after alcohol condensation.

The powdery culture sup preferably comprises at least one of a growth factor selected from the group consisting of platelet derived growth factor (PDGF), vascular endothelial growth factor (VEGF), insulin-like growth factor (IGF), keratinocyte growth factor (KGF), hepatocyte growth factor (HGF), and transforming growth factor (TGF).

Furthermore, the cosmetic has preferably any one of form selected from the group consisting of a liquid, a cream, an ointment, a gel, an emulsion, and a cataplasm.

The cosmetic is preferably applied for skin including scalp or hair.

The second aspect of the present invention is a cosmetic stored in a container comprising: a first compartment, a second compartment, a bar member to form a through hole on a partition between said first and said second compartment; wherein, said first compartment includes a solvent; said second compartment includes an active ingredient composition comprising a carrier and any one of powdery culture sup selected from the group consisting of that of a swine dental pulp exfoliated deciduous teeth, that of a swine bone marrow, and that of a swine adipose tissue; and said bar member formed said through hole in said partition to solve said active ingredient composition into said solvent immediately before to use said cosmetic. The solvent is preferably any one of liquid selected from the group consisting of a liquid to which a minus ion, anion, is charged, saline and phosphate buffered saline.

The cosmetic is preferably applied for skin including scalp and hair.

The third aspect of the present invention is an iontophoresis method for a protein comprising the steps of: placing a sheet form of a moisture-retaining member containing said cosmetic as described above on a predetermine site through which said cosmetic is absorbed; attaching a positively-charged electrode to predetermined site; and attaching a negatively- charged rod-like electrode to rotate and move on said sheet from.

The predetermined site is preferably any one selected from the group consisting of an arm, a hand, a palm, a leg, and a ham, and a bottom of foot.

The fourth aspect of the present invention is a dermal formation promoting agent comprising a powdery culture sup of a stem sell selected from the group consisting of that of a dental pulp stem cell, a bone marrow stem cell, and an adipose stem cell of non-human mammal. Here, the stem cell of the non-human mammal is preferably obtained from any one of tissue selected from the group consisting of the swine dental pulp tissue of exfoliated deciduous teeth, the swine bone marrow tissue, and the swine adipose tissue. The powdery culture sup is preferably prepared from the culture sup selected from the group consisting of the dental pulp of the exfoliated deciduous teeth, the bone marrow, and the adipose tissue by freeze-drying after alcohol condensation.

The powdery culture sup preferably comprises at least one of a growth factor selected from the group consisting of platelet-derived growth factor (PDGF), vascular endothelial growth factor (VEGF), insulin-like growth factor (IGF), keratinocyte growth factor (KGF), hepatocyte growth factor (HGF), and transforming growth factor (TGF).

The cosmetic preferably has any one of the form selected from the group consisting of a liquid, a cream, an ointment, a gel, an emulsion, and a cataplasm.

### Advantageous Effect of the Invention

According to the present invention, the cosmetic having advantageous effects such as line smoothing, whitening, new hair growing, making hair thick and the like.

### Brief description of the drawings

Fig. 1 is a schematic drawing showing the dermal regeneration effect by using the culture sup of the swine stem cell to skin aging (dermal aging by light) by UV irradiation.
Fig. 2 is a graph showing the ratio of the bromodeoxyuridine (BrdU) uptake cells, when BrdU incorporation into the swine bone marrow stem cells (pBMSC), the swine dental pulp stem cells (pDPSC) and the swine exfoliate deciduous teeth stem cells (pSHED) are allowed. In Fig. 2, * shows p < 0.05.
Fig. 3 is a microscopic image showing the effects of the swine growth factor (pGF) by the photoaged skin. Fig. 3(A) shows the microscopic observation image of a skin replica formed from a control skin fragment to which pGF was not administrated; and Fig. 3(B) is that of a sample skin fragment to which pGF was administrated.
Fig. 4 is the graph showing skin aging status of the control group to which the culture sup of the stem cell was not administrated; the group to which the culture sup of the swine exfoliate deciduous teeth stem cells (pSHED-CM) is administrated; and the group to which the swine exfoliate deciduous teeth stem cells (pSHED) is administrated.
Fig. 5 is a histologically-stained image showing the skin status of the each group mice shown in Fig. 4. In the Figure, upper arrow shows the epidermis and the lower arrow shows dermis, respectively.
Fig. 6 is the graph respectively showing the cutaneous cells without UV irradiation (negative control), with UV was irradiation (UVS, photoaged cutaneous cells), and with both of photoaging and subsequent the swine growth factor (PGF) administration. In Fig. 6, ** means p < 0.01.
Fig. 7 is the microscopic image respectively showing the status of the cells the cultured swine bone marrow stem cells (pBMSC) and the swine dental pulp stem cells (pSHED) on day 3 and day 7.
Fig. 8 is a photograph showing the status of the blotches and the spots. Fig. 8(A) shows the status of the blotches and the spots before test starting, and Fig 8(B) shows those of after the test finishing (6 month later).
Fig. 9 is the photograph showing the wrinkle on a forehead before and after the test. Fig. 9(A) shows the status of the wrinkle before test starting, and Fig 9(B) shows that of after the test finishing (12 month later).
Fig. 10 is the microscopic images of the replicas made of the skin prepared as the same as those in Fig. 3 before and after the test. Fig. 10(A) shows the status before the test starting, and Fig. 10(B) shows that after the test finishing.
Fig. 11 is the photograph showing the status of the hair before the test starting.
Fig. 12 is the photograph showing the status of the hair after 14 days of the test starting.
Fig. 13 is the photograph of the scalp before the test starting.
Fig. 14 is the photograph of the scalp on day 3 from the test starting.
Fig. 15 is the photograph of the scalp on day 5 from the test starting.
Fig. 16 is the photograph of the scalp on day 7 from the test starting.

### Mode for carrying out the invention

The present invention is explained more detail in below.

The powdery culture sup of the swine dental pulp stem cell used for the cosmetic of the present invention is used as follows.

Firstly, as a swine jaw, it is preferably to use a lower jaw with tooth immediately after slaughtering for meat. Here, since obtained cells are fresh, the jaw from the swine immediately after slaughtering is preferable. Also, the jaw of the swine for meat is preferable: because their dental pulps may be easily obtained; they are highly safe without infections to viruses or bacteria; and is low in cost. It is also preferably used the swine of 3 to 12 month old from the view point of stem cell number, and is more preferably used that of 5 to 6 month old.

Firstly, the lower jawbone with tooth of the swine for meat immediately after slaughtering are refrigerated from the temperature range of -40 to -30 °C to be transported. For example, an icebox including refrigerants (-30 °C) and the like may be used.

Next, in order to sterilize the entire surface of the lower jaw, for example, disinfection is performed by using an antiseptic agent such as Isodine (registered trademark) and the like. Then, a crown of the tooth is cut in horizontal direction by using, for example, a diamond point for a dentist and the like. Subsequently, it is cut in vertical direction along with a pulp space. By these cuttings, the overcanopy of the tooth may be smoothly removed. Then, the dental pulp is collected both from the dental crown and dental root portions treated as mentioned above by using, for example, a hand scaler for the dentist, a hand file for the dentist and the like.

Obtained dental pulps are chopped by using an ophthalmic knife to be suspended in a basal medium containing predetermined serum and antibiotics. As the basal medium used here, Dulbecco's modified eagle's MEM containing 5 to 20% (v/v) of bovine serum, and 1 % (v/v) of 5 x 10³ to 5 x 10⁴ U/mL of penicillin and 1 % of 5 to 50 mg/mL of streptomycin, and the like.

Next, an enzyme solution containing predetermined concentration of both collagenase and disperse is prepared to separate dental pulp cells into single cells. For example, obtained cells are suspended in the enzyme solution containing both of 1 to 5 mg/mL of collagenase and disperse to be treated at 35 to 38 °C for 30 minutes to 1.5 hr in a thermostatic chamber.

After that, they are centrifuged for 2 to 5 minutes, preferably, for 3 minutes, (for example, about 1,500 rpm (about 1,000 x g)); the isolated dental pulp cells by the enzyme treatment are collected. In this time, it is preferable to avoid the use of a cell strainer for cell sorting, because it decreases the recovery ratio of a neural stem cell fraction for SHED or DPSC.

Also, the adipose cell may be obtained as follows. Since the cells are fresh and easily obtained, the adipose tissue in the swine mesentery is used. The transport conditions are the same as those used for the swine lower jaw with tooth for meat. Firstly, the adipose tissue of the swine mesentery for meat immediately after slaughtering is excised and collected. Next, other tissues attached to the obtained adipose tissues are removed, and then the adipose tissue is minced by using surgical knife or scissors.

Next, the minced tissue is suspended in the basal medium containing the predetermined serum, antibiotics and the like. The basal medium used here is as mentioned above. Subsequently, as the same as the preparation of the dental pulp stem cells, the enzyme solution containing both of collagenase and disperse at the predetermined concentrations are prepared as mentioned above to be used to obtain the adipose stem cells.

Next, each of the isolated dental pulp stem cells, bone marrow stem cells or adipose cells are cultured by using predetermined media to obtain the culture sups thereof. For example, the obtained cells are re-suspended in 2 to 8 mL of the basal media to be plated to suitable size of dishes for adherent cell cultures. For example, the suspension is plated into the dishes with 6 cm diameter, and the culture medium (for example, 10%FCS containing DMEM (Dulbecco's Modified Eagle's Medium)) is added into the dishes. After that, they are cultured for about 10 to 20 days in the presence of 5% CO₂ in an incubator adjusted at 35 to 38 °C.

After removal of the medium, the cells are washed from 1 to several times with PBS and the like. Instead of above-mentioned procedure (the removal of the medium and wash of the cells), the adherent cells which formed colonies may be collected. In this case, for example, the cells are treated by using a solution including both of 0.01 to 0.1 % trypsin and 2 mM EDTA for 5 minutes at 37 °C to be detached from the dish. By collecting the detached cells, adherent stem cells may be obtained.

Subsequently, selected adherent cells are cultured. For example, the cells are plated to similar dishes for the adherent cell culture, cultured under the similar conditions, and passed depending on the necessity. For example, when the cells become sub-confluent (about 70 % of the bottom area of the culture vessel is covered by the adherent cells) or confluent by macroscopic observation, the cells are detached from the culture vessel by using the same treatment as described above to be collected. Then, they are again plated in the culture vessel including the fresh medium having the same composition. Passage may be performed repeatedly until the cell number becomes sufficient. For example, when the passage culture is repeated 1 to 8 times, the cell number reaches about 1 x 10⁷ cells /mL.

After the above-mentioned cultivation, the cells may be collected, and, for example, stored in liquid nitrogen.

In the initial stage of culture, the medium is replaced as necessary to reach sub-confluent, for example, 2 to 3 times a week. The sup of the sub-confluent flask is removed by using an aspirator and the like; then, Hepes buffer including 0.01 to 0.1 % (v/v) of trypsin is poured into the flask, the cells are detached from the bottom of the flask. Fresh medium is added into the flask in a suitable amount to suspend the detached cells to transfer into a sterilized centrifuge tube. Next, the tube is centrifuged at 800 to 1,200 x g (about 1,500 rpm) at room temperature for 2 to 5 minutes, preferably for 3 minutes. For example, Hepes buffer including 0.05 % of trypsin is prepared, and add the flask, form which the medium was removed, in a small amount so as to penetrate entire of the bottom of the flask. When the cells are detached from the bottom, about 5 to 8 mL of fresh medium is added. Then, the medium including the cells may be centrifuged at 1,500 rpm for 3 minutes at room temperature to be concentrated. The cell concentration in the condensed solution per mL is obtained by using viable count method; then, 10 to 40 mL of them are added into the flask including about 10 to 40 mL of fresh medium to perform passage culture to obtain the exfoliated deciduous teeth dental pulp stem cell (pSHED), the bone marrow stem cell or adipose cells of swine.

Also, a hole is formed in a lingual side of a lower anterior tooth of the swine lower jaw bone body by using, for example, the diamond point for the dentist and the like. For example, a syringe with a needle of 18G is inserted into the hole to aspirate. The obtained bone marrow is cultured until it becomes sub-confluent in the culture flask as mentioned above. Then, the sub-confluent cells are detached from the bottom of the culture flask, and separated by the centrifugation. The separated cells are passed to obtain the swine bone marrow cells (pBMSC).

The stem cells obtained as described above are the deciduous teeth dental pulp stem cells, the bone marrow stem cells or adipose stem cells, which are mesenchymal-derived somatic stem cells. In the culture sups of these stem cells, cytokines such as vascular endothelial growth factor(VEGF), hepatocyte growth factor (HGF), insulin-like growth factor (IGF), platelet-derived growth factor (PDGF), transforming growth factor -beta (TGF-β)-1 and -3, TGF-α, KGF, HBEGF, SPARC, and others are secreted. Therefore, these stem cells are preferably employed.

The stem cell for obtaining the culture sup of the stem cell used in the present invention is preferably obtained from the swine for meat; because the cells themselves are highly safe, they neither have any surgical invasive matters caused by removal from the human tissues; nor ethical matters for use thereof. Therefore, there are great advantages.

Also, the culture sup of the stem cell (pSHED-CM) preferably comprises a combination of at least two factors selected from the group consisting of VEGF, HGF, IGF, PDGF and TGF-β as mentioned above, because of the excellent collagen regeneration ability.

Note that the stem cell culture sup may be used by the addition of at least one known corresponding cytokine.

The stem cell culture sup is obtained by culturing the stem cell under the predetermined conditions, and removing the cells from the medium with the centrifugation and the like. Thus obtained culture sup is subjected to several treatments such as, for example, the centrifugation, condensing, replacing the solvent, dialysis, lyophilizing, desalting and others, and the treated culture sup may be used. For example, lyophilized powder of the culture sup is obtained by freezing the culture sup in dry ice-acetone and then drying.

As the medium for the stem cell culture, either the sole basal media or the basal media supplemented with serum and the like may be used. As the basal media, the following media may be used: DMEM, Iscove's Modifed Dulbecco's Medium (IMDM; GIBCO, etc.), Ham's F12 medium, HamF12; SIGMA, GIBCO, etc.), RPMI1640 medium, and the like. Also, a mixed media comprising at least two media may be used. As an example of the mixed medium, there is mentioned that including IMDM and HamF12 in equal amount (for example, it is commercially available under the product name: IMDM/HamF12 (GIBCO)).

As the components to be added to the media, there are mentioned, for example, serum (fetal calf serum, fetal equine serum, human serum, and sheep serum, etc.), serum replacement (Knockout serum replacement (KSR), etc.), bovine serum albumin (BSA), antibiotics, vitamins, minerals, and the like.

Note that serum-free media are preferably used through entire steps of the stem cell culture, or in several passage cultures, at least in the last passage culture, for obtaining "the stem cell culture sup" without serum as described above. The stem cell culture may be performed by applying the generally employed conditions.

As mentioned above, pSHED included in the cosmetic of the present invention comprises a variety of growth factors. Therefore, such growth factors may be absorbed transdermally by applying it onto the skin. As the components to be added to the cosmetic of the present invention, there are mentioned, for example, organic bioabsorbable materials such as hyaluronic acid, collagen, fibrinogen (for example, BOLHEAL (registered trademark)) and the like; highly biocompatible gelation materials such as hyaluronic acid, collagen, fibrin glue, and the like. Alternatively, pharmaceutically available other components (for example, a carrier, an excipient, a disintegrator, a buffering agent, an emulsifier, a suspending agent, a soothing agent, a stabilizer, a preservatives, an antiseptic, physiological saline, etc.) may be added.

As collagen used here, soluble collagen such as acid-soluble collagen, alkaline-soluble collagen, enzyme-soluble collagen and the like may be preferably used, because of their compatibility to the cosmetic of the present invention.

As the excipient, for example, lactose, starch, sorbitol, D-mannitol, sucrose and the like may be used. As the disintegrator, carboxymethyl cellulose, calcium carbonate and the like may be used. As the buffering agent, phosphate, citrate, acetate and the like may be used. As the emulsifier, gum arabic, sodium alginate, tragacanth gum and the like may be used. As the suspending agent, glyceryl monostearate, aluminium monostearate, methyl cellulose, carboxymethyl cellulose, hydroxymethyl cellulose, sodium dodecyl laurate and the like may be used.

As the stabilizer, propylene glycol, ascorbic acid and the like may be used. As the preservative, phenol, benzalkonium chloride, benzyl alcohol, chlorobutanol, metylparaben, and the like may be used. As the antiseptic, benzalkonium chloride, para-hydroxybenzonate, chlorobutanol and the like may be used. Other than those, pH control chemicals may be included.

The final cosmetic form of the present invention is not particularly limited, and they may be the skin toning lotion, milky lotion, lotion, gel, cream and the like.

Also, the cosmetic is preferably applied to the skin including scalp and hair.

The preparation method of the cosmetic as mentioned above is not particularly limited. For example, when the swine lower jay is used as the raw material, it is preferably prepared as describe below.

Firstly, the dental pulp stem cells are obtained from the swine lower jaw obtained as described above. The swine adipose tissue is used instead of the swine lower jaw; the adipose stem cells are obtained as described above. Next, for example, the dental pulp stem cell is cultured under the above-mentioned conditions by using the serum-free medium, and then their culture sup is collected by using, for example, a syringe, a pipette and the like. The collected culture sup may be used as is, or used after at least one of the following treatment such as the centrifugation, condensation, dialysis, lyophilization, dilution, and desalting.

Note that as shown in the following examples, the culture sup of the stem cell shows predetermined actions without highly purification. This means that the composition used for the cosmetic of the present invention may be prepared conveniently and speedy. Therefore, there is a merit that it is avoidable to decrease the component activities during the purification.

The culture sup obtained as mentioned above is firstly determined its protein contents. Then, its collagen purification activity is determined to obtain specific activity. By using the specific activity as an indicator, the quality of the product may be maintained constant.

If the specific activity is low, the sup may be condensed by using a spin column condensation or ethanol precipitation. In the spin column condensation, the maximum volume of the culture sup for the predetermined size of the spin column is applied to the column; then the column is subjected to the centrifugation at 3,000 x g to 5,000 x g for about 30 to 90 minutes. The serum-free medium is added into the tube in the equal volume to the obtained condensed culture sup volume; then, the tube is subjected to the centrifugation at 3,000 x g to 5,000 x g for about 30 to 90 minutes. By this operation, the media of the culture sup is replaced to the serum-free media.

For example, Amicon Ultra Centrifugal Filter Units-10K (Millipore) is used as the spin column; the maximum volume to be loaded on the column is 15mL. Therefore, about 15mL of the culture sup is loaded to the column, and then column is centrifuged in 4,000 x g for about 60 minutes at 4 °C. By this operation, it gives up to 200 µL of the condensed culture sup. The same volume of the sterilized PBS as that of the culture sup, which is condensed up to 200 µL, is added, and again the tube is centrifuged 4,000 x g for about 60 minutes at 4 °C to replace the solvent of the culture sup to PBS. The obtained solution is collected into a micro test tube, and it is used as the condensed stem cell culture sup. If the finally obtained volume is about 200 µL, the enrichment of the sup is 75 fold.

For example, ethanol precipitation is performed as follows. Firstly, 9 times volume of 100 % ethanol is added to a certain amount of the culture sup in a tube to mix, and then the mixture is stood at about -10 to 30 °C for 30 to 90 minutes. Next, the tube is centrifuged at about 4 °C in 10,000 to 20,000 x g for about 10 to 20 minutes. The sup is removed, and then 1/5 volume of 90 % of ethanol is added, and then agitated well. Subsequently, it is centrifuged at about 4 °C, in 10,000 to 20,000 x g for 3 to 10 minutes. The sup is removed, and the obtained pellet is suspended in the predetermined volume of the sterilized water, for example, 500 µL of the sterilized water to be collected into the micro test tube. Thus, the condensed sup of the stem cell may be obtained.

For example, 45 mL of 100 % ethanol is added to 5 mL of the culture sup in a tube to be mixed, and then it is stood at -20 °C for 60 minutes. Then, it is centrifuged at 4 °C in 15,000 x g for 15 minutes, and then the sup is removed. After that, 10 mL of 90 % ethanol is added and agitated well. Again, it is centrifuged at 4 °C in 15,000 x g for 5 minutes, and the sup is removed. The obtained pellet is suspended in 500 µL of the sterilized water to be collected into the micro test tube. Thus, the condensed culture sup is obtained. In this case, the enrichment of the sup is 10 fold, if the first volume of the sup used is 5 mL.

The culture sup of the stem cell used to the cosmetic of the present invention may be lyophilized product as follows. Firstly, the culture sup or the condensed one obtained as mentioned above is dispensed into each container with lid of which volume is about 50 to 150 mL in a fixed amount. Then, the lid of the sample tube is tightly sealed, and the tube is frozen at about -100 °C to about -60 °C for 2 hr to half day. After the sup is frozen, the tube is opened to be set in a lyophilizer. Next, the sample is lyophilized for 1 to 2 days to obtain the lyophilized stem cell culture sup, which may be stored at about - 100 °C to -60 °C.

For example, the culture sup is respectively dispensed into 8 vials with rubber stopper, of which volume is 50 mL, by 20 mL. Then, the vials are sealed with the rubber stoppers and frozen at about -30 °C over a period of hours. Next, the rubber stoppers are removed, and the vials are set in the lyophilizer (Yamato Science Corp., DC41A). Next, the lyophilization is continued to dry up the sample. By this, the lyophilized culture sup may be obtained.

According to the above-mentioned procedures, the lyophilized products having excellent preservation stability is produced.

When the cosmetic is introduced through the skin by using the iontophoresis, the cosmetic is absorbed in the sheet formed moisture-retaining member, and the member is located on the site through which the cosmetic is introduced. Then, the positively-charged electrode is attached to the predetermined portion, and negatively-charged rod-shaped electrode is rotated and moved on the sheet member.

Here, the predetermined site is not particularly limited. However, the site is preferably selected from the group, for example, consisting of arm, hand, palm, leg, ham, and foot bottom, because the positively-charged electrode may be stably attached to such sites.

As the sheet formed moisture-retaining member, a commercially available sheet for pack made of unwoven cloth, gauze, cosmetic cotton, and the like may be used. If the cosmetic is a liquid form or milky lotion form, they may be used to impregnate the sheet formed moisture-retaining member. Alternatively, if the cosmetic is cream or gel, it may be applied to the skin and covered by the sheet formed moisture-retaining member.

In the following, the present invention is explained as the example, when it is the skin toning lotion. Firstly, the powdery culture sup is dissolved in proper amount of the saline to prepare a powder-dissolved solution. The moisture-retaining member is impregnated into that so as that the solution is dripped. Next, the moisture-retaining member is attached to the predetermined portion of the body, for example, entire of the face, and a part of the body is attached to the positively-charged electrode. For example, the positively-charged electrode is grabbed by one hand.

Next, the negatively-charged electrode in placed on the moisture-retaining member, and then slowly rotated and moved on the member. It is not necessary to apply pressure. Since all of the growth factors included in the solution are negatively charged, they are repelling to the negative charge on the roller, and transfers to the positively-charged electrode. By this transfer, the growth factors are absorbed from the skin.

Alternatively, the cosmetic may be stored in a bottles made of plastic or glass, the bottles made of plastic or glass with a dropper, a container having thin outlet made of plastic and the like to be used. When it is used as the cosmetic for scalp or hair, the container having thin outlet made of plastic is preferably used, because the cosmetic is attached to the scalp.

The cosmetic is attached to the scalp and hair to be coated evenly. After that, it is introduced by iontophoresis method through the skin as described above. Then, entire of the hair is wrapped by using a plastic film, for example, polyethylene film and the like to be packed.

For both case described above, a steam-generating unit is placed to a predetermined distance from the head so as to spray the steam, it provides high introduction efficiency.

Herein below, the example of the present invention is explained; however, the present invention is not limited to them. Also, the term, "%" in the example is weight (mass) basis, unless otherwise noted.

The present invention is not limited to the description of the above-mentioned embodiment or the examples. If they are fallen in the scope of the claims, and various modifications of them are predictable by the person skilled in the art, they are contained in the present invention.

### Example 1

Production and quality evaluation of the growth factors derived from the swine SHED/BM/adipose tissue

### <Materials and method>

### (1) Fractionation and culture of the cells

From Shokuniku Kosha Co. Ltd (Minato-ku, Nagoya city, Japan), the jaw (the lower jaw with tooth) and mesentery of 5 to 6 month of swine were obtained. Those immediate after slaughtering were transported in the ice box including the thermal gel (-30 °C).

From the swine tooth and the lower jaw, the swine dental pulp stem cells (SHED) were obtained according to the following procedure.

The transferred swine tooth and the lower jaw were sterilized with Isodine. Then, the crowns of the tooth in the lower jaw were cut in horizontal direction by using the diamond point for the dentist, and cut in the vertical direction along with the pulp space to delete the overcanopy. The dental pulps were collected from the crowns and roots of the tooth treated as described above by the scaler for the dentist.

The obtained dental pulps were chopped by using the ophthalmic knife to be suspended in 2 mg/L of collagenase solution. The solution was placed in the incubator at 37 °C for 1hr, and the cells were separated. In order to obtain the cells for passage, the separated cells were preliminarily cultured in Dulbecco's modified Eagle's MEM (DMEM; SIGMA, St. Louis, MO) supplemented 10 % FBS and 1 % Anti-Anti (Invitrogen, Carlsbad, CA) under the conditions at 37 °C and 5 % CO₂.

Firstly, in the initial stage of the culture, the cells were cultured until sub-confluent, replacing the medium 2 to 3 times per week. The sub-confluent cells were detached from the flask by using Hepes solution including 0.05% trypsin, and then, the cells were collected by the centrifugation in 1,500 rpm for 3 minutes at room temperature. The obtained cells were transferred into the fresh medium and the entire of the cells were used to the passage culture under the same conditions as described above.

The swine bone marrow (BM) was obtained from the cortical bone of the lower jaw body. Firstly, the hole was formed in the lingual side of the lower anterior tooth of the swine lower jaw bone by using the diamond point for the dentist. The 5 mL of the syringe with the needle 18G was inserted into the hole to aspirate the bone marrow to collect. The collected bone marrow was transferred into the DMEM supplemented with 10 % bovine serum, 100 U/mL of penicillin and 100 µg/mL of streptomycin; then they were preliminarily cultured under the same conditions. Then, they were cultured under the conditions at 37 °C and 5% CO₂, until they became sub-confluent. All of the supplemented amounts as described were shown at the final concentrations.

Similarly to the swine SHED, the cells were detached by using 0.05 % trypsin solution to be centrifuged for 3 minutes in 1,500 rpm to be subjected to the passage culture.

From the swine mesentery, the adipose tissues were excised by using the dissecting scissors and knife to remove unnecessary tissue parts, and washed out the blood in the phosphate buffered saline. Except those, the adipose cells were separated into the single cell as described above to obtain the adipose stem cells.

### (2) Analysis of the cell growth

According to the instruction attached BrdU staining kit (Invitrogen, Carlsbad, CA), bromodeoxyuridine (BrdU) was incorporated into SHED or BM obtained as described above. Then, the growth speed for 12 hr of each cell was evaluated. For the sample of each group, three slide glasses were prepared for one sample to evaluate in triplicates. The experiments were repeated 5 times. After one-way analysis of variance, Turkey-Kramer test (Tukey-Kramer test) was employed to perform significance test.

In order to perform STRO-1 immunofluorescence staining, the swine SHED or the swine BM was fixed by using 3 % paraformaldehyde. After that, they were rinsed twice with the phosphate buffered saline (PBS), treated with 100 mM glycine solution for 20 minutes. Next, these cells were permeabilized with 0.2 % Triton-X (Sigma-Aldrich, St. Louis, MO) solution for 30 minutes at room temperature. Then, PBS including 5 % Equus asinus serum and 0.5 % bovine serum albumin was added to the cells, which were incubated at 37 °C for 20 minutes in the solution.

Next, the primary antibody, mouse anti-human STRO-1 antibody (R&D, Minneapolis, MN) and cells were mixed at the ratio of 1: 100, and incubated for 1 hr at room temperature in PBS. Next, PBS was removed by the aspiration, the secondary antibody, goat anti-mouse immunoglobulin M-FITC antibody (Southern Biotech, Birmingham, AL) and the cells were mixed at the ratio of 1: 500, and incubated for 30 minutes at 37 °C. Then, they were mounted by using the vector shield and DAPI (Vector Laboratories Inc, Burlingame, CA).

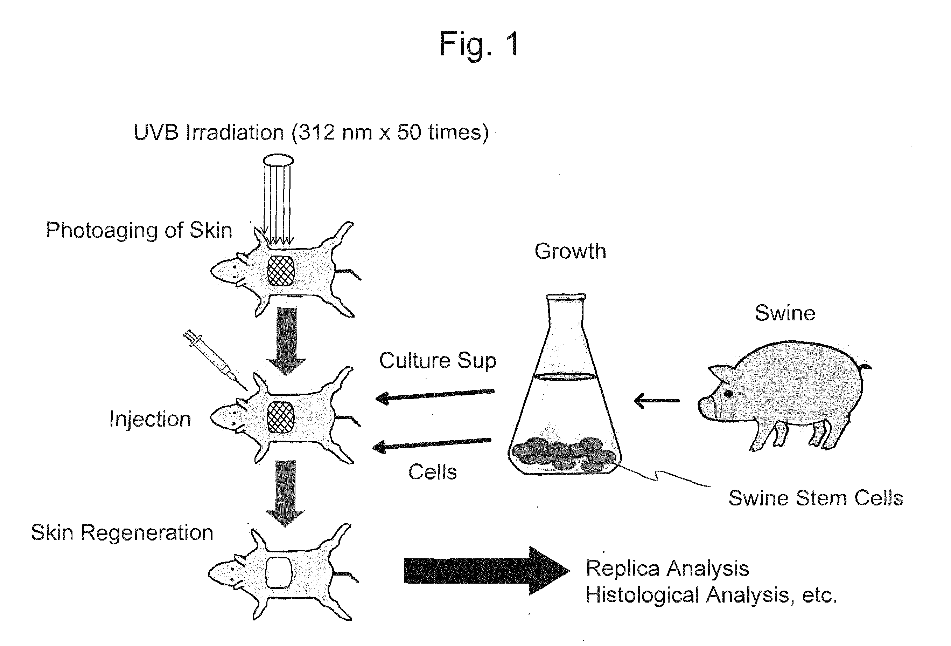

### (3) Animal experiment (see Fig. 1)

Five week age of female hairless mice (Hos; HR-1) were provided by SLC (SLC Inc., Shizuoka, Japan). The all of the mice were placed under the conditions controlled temperature, humidity (22 ± 1 °C, 50 % humidity) and 12 hr light dark cycle. The animals could freely take water and solid feed, and they could freely move in the cage during the radiation exposure. Observation was carried out every day. With UVB irradiation device RMX-3W (Handok Biotech, Seoul, Korea), UVB was irradiated onto the mouse back for 10 weeks at 5 times a week, and the distance from the lump to the animal back was 89 cm. The irradiation was performed by using an array composed of 10 SE lump (Toshiba) without filtering for UVB. Peak irradiation wave length was about 312 nm. Also, the irradiation amount was set to that the radiation ray having the wavelength of 290 to 320 nm occupies 55 % of whole amount of UVB.

The irradiation amount was set to 1 MED (minimal erythema dose; 60 mJ/cm²) for the first 2 weeks, 2 MED (120mJ/cm²) for 3^{rd} week, 3 MED (180 mJ/cm²) for 4^{th} week, 4 MED (240 mJ/cm²) for 5^{th} to 8^{th} weeks. Total UVB radiation dose was about 115 MED (6.9J/cm²) to induce the wrinkle formation.

At five weeks after the wrinkle induction, pSHED-CM (100 %) was administrated in s.c. to the limited area of the mouse back. As the positive control, the swine SHED (4 x 10⁵) suspended in PBS was directly injected into the dermis, and as the negative control, PBS was solely directly injected into the dermis.

### (4) Preparation of the swine SH-CM

The swine SHED (4 x 10⁵ cells) was cultured in DMEM/F12 (Invitrogen-Gibco-BRL, Grand Island, NY) serum free medium at 37 °C, under 5% CO₂. After 72 hr, the culture sup of the swine SHED was collected to be centrifuged in 300 x g for 5 minutes at room temperature. Then, it was filtered by using a filter having the pore size of 0.22 mm (Millipore).

### (5) Analysis of skin replica and its image

At wrinkle induction and after 1 week from the above-mentioned injections, a negative type replica was prepared by using a silicone rubber impression material (Flextime 1; Heraeus Kulzer, New York, NY) from the skin surface of the mouse back. In order to obtain a wrinkle replica from the same area of the skin, a permanent marker was used to mark up the area.

After 5 weeks from the final injection of either the swine SH-CM or SHED to the skin, the impression was obtained from the marked up area. For easy measurement, all replicas were cut into 1 cm on four sides, and the back side of the replica was processed to be flat face by using the same impression material. Shedding the light at an angle of 208 °, the images of the replicas were captured by using a CCD camera.

The image of the negative type replica was observed by using the wrinkle analyzer, Skin visiometer (skin visiometer) SV 600 (Courage & Khazaka, Cologne, Germany). Parameters used for the evaluation of the skin wrinkle were the number and the depth of the skin wrinkle per unit area, and the area of the wrinkle.

### (6) Histological analysis

The back skin (1 cm x 1 cm) was fixed in the neutral buffer including 10 % formalin. Then, the fixed sample was embedded into the polyester wax to prepare a section having 6 mm thickness. The section was exposed to hematoxylin & eosin (H & E) to perform Masson trichrome staining.

Firstly, hematoxylin & eosin (herein below, it is referred to as "H&E staining") was carried out. The section was subjected to deparaffinization by using 3 batches of Xylene (Resomoll). Then, it was subjected to dehydration by using 4 batches of absolute ethanol, and washed with running water for 3 minutes. Next, it was immersed in Mayer's hematoxylin solution for 5 minutes to stain the nucleus, and then washed with running water of about 45 °C for 3 minutes.

Next, it was immersed in the eosin solution for 5 minutes. Then, the tissue was dehydrated by using 4 batches of absolute ethanol, and treated 4 timed with xylene to softly mounted to remove the colorant stayed in other positions, which was not to be stained.

Subsequently, it was subjected to Masson trichrome staining. The section was subjected to deparaffinized as the same as that for hematoxylin & eosin staining, and then washed. The first mordanting was performed by using the mixed solution of equal volume of 10 % trichloroacetate (Wako Pure Chemicals, special grade) solution and 10 % potassium dichromate (Wako Pure Chemicals, special grade) solution. Next, the section was washed with water for 3 minutes and then distilled water for 1 minute.

A solution including 1.0 g of hematoxylin (MERCK) and 100 mL of 95 % ethanol and B solution including 2 g of ferric chloride (Wako Pure Chemical, Special grade, in both), 1 mL of hydrochloride and 95 mL of distilled water were mixed when using to prepare Weigert's iron hematoxylin to be used for immersing the section for 10 minutes. Next, it was washed with water for 10 minutes.

Equal volume solutions of 2.5% phosphotungstic acid (Wako Pure Chemicals, special grade) and 2.5% phosphomolybdic acid (Wako Pure Chemicals, special grade) were mixed, and the section was immersed for 1 minute therein to perform the second mordanting. Next, the section was immersed in 0.75% Orange G (Wako Pure Chemicals, 1^{st} grade) solution for 2 minutes. Then, it was subjected to treatment with 2 batches of 1 % acetic acid solution.

Next, ponceau de xylidine - acid fuchsin solution was prepared by mixing 2 volumes of 1% of ponceau de xylidine (CHROMA) in 1 % acetic acid solution and 1 volume of 1 % acid fuchsin (Wako Pure Chemicals, chemical grade) in 1 % acetic acid solution. Then, the section was immersed in the solution for 20 minutes. Then, it was subjected to treatment with 2 batches of 1 % acetic acid solution.

Subsequently, the section was immersed in 2.5 % phosphotungstic acid for 7 minutes, and then it was subjected to treatment with 2 batches of 1 % acetic acid solution. Next, it was stained by using aniline blue and subjected to treatment with 2 batches of 1 % acetic acid solution. Then, it was immersed in isopropanol one by one to perform to remove the colorant stayed in other positions, which was not to be stained as the same as those done in hematoxylin & eosin staining.

### (7) The culture of dermis fibroblast (HDF) in human dermis and irradiation amount of UVB

HDF was cultured in DMEM supplemented with 10 % fetal bovine serum, 100 U/mL of penicillin, and 100 mg/mL of streptomycin under 5 % CO₂ at 37 °C. The cells in the culture flask were cultured in the serum-free medium for 24 hours. After that, they were washed with PBS, and subjected to UVB exposure with 3 to 4 drops of PBS, wherein the irradiation amount of cells were between the ranges of 50 to 250 mJ/cm². UVB irradiation was performed by using the above-mentioned UV source (Waldmann, Schwenningen, Germany). Immediately after the irradiation, PBS was aspirated, and was replaced with the complete medium. UVB irradiation amount was finally fixed to 70 mJ/cm², and it was used for following experiments.

### (8) Cell growth assay

HDF was plated at 5 x 10³ cells /well in a 96 well plate, and their growth rate was measured by using CCK-8 kit (Dojindo, Gaithersburg, MD). They were cultured in the serum-free medium for 24 hours; subsequently, they were continue to culture further 24 hours with or without the swine SH-CM. Then, they were exposed to UVB (70mJ/cm²) for 90 seconds.

Subsequently, UVB-irradiated cells were cultured for 24 hours in the complete medium to be collect. HDF-F was poured into 10mL of CCK-8 solution, and then incubated for 3 hours. By using the microtiter plate reader (TECAN, Gro" dig, Austria), absorbance at 450 nm was measured.

On the basis of the calibration curve prepared by using the control group, cell number was obtained from OD values of each well.

### (9) Western blot analysis

HDF (2 x 10⁴ cells/well) were plated in a 24 well plate, and they were the pretreated as described above. Next, the cells were lysed in RIPA buffer (50mM Tris-HCl (pH 7.4) including 0.15M NaCl, 1 mM EDTA, 1% Triton X-100, 1 % SDS, 50mM NaF, 1 mM Na₃VO₄, 5 mM dithiothreitol, 1 mg/mL leupeptin, and 20 mg/mL PMSF). The cell-lysed solution was determined by using Biuret reagent, and 50µg of protein was subjected to 8 % SDS-polyacrylamide gel electrophoresis (SDS-PAGE). After finishing SDS-PAGE, the gel electrophoresis pattern was transferred to PVDF membrane.

The membrane was incubated for 15 minutes at room temperature together with and anti-type I collagen antibody (Santa Cruz, Saint Louis, MO) and an anti-matrix metalloproteinase 1 (MMP-1) antibody (Calbiochem, Darmstadt, Germany). Next, the PDF membrane was washed by using PBS, and then it was incubated for 15 minutes at room temperature together with anti-goat IgG conjugate with horse radish peroxydase (1: 100,000, Santa Cruz, Saint Louis, MO). After that, it was blotted with immunoglobulin Western reagent. X-ray film was laid over the membrane to be exposed.

### <Results>

### (1) Feature analysis pSHED and pBMSC

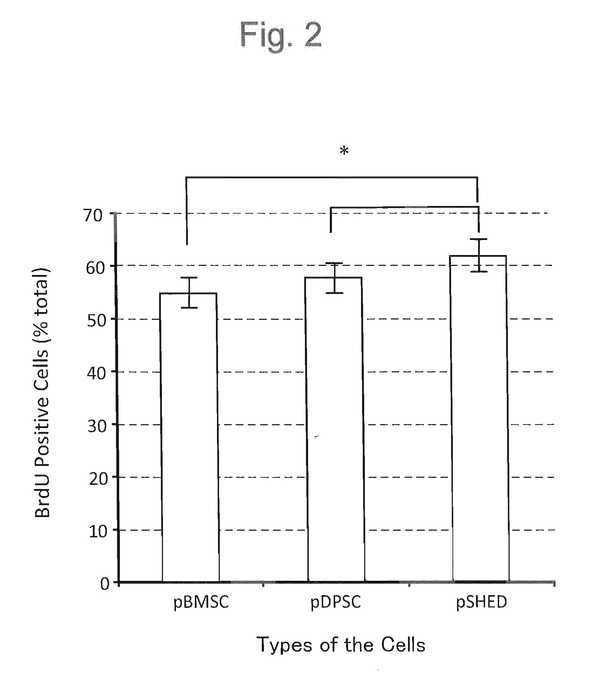

Both pSHED and pDPSC showed the same fibroblast shapes as those of pBMSC. From the immunofluorescence analysis, it was demonstrated that both pSHED and pBMSC contain STRO-1 positive cells. Also, it was shown that the growth rate of pSHED was significantly higher than that of pBMSC (see Fig. 2).

### (2) Reduce of UV induced wrinkle with the swine SHED-CM

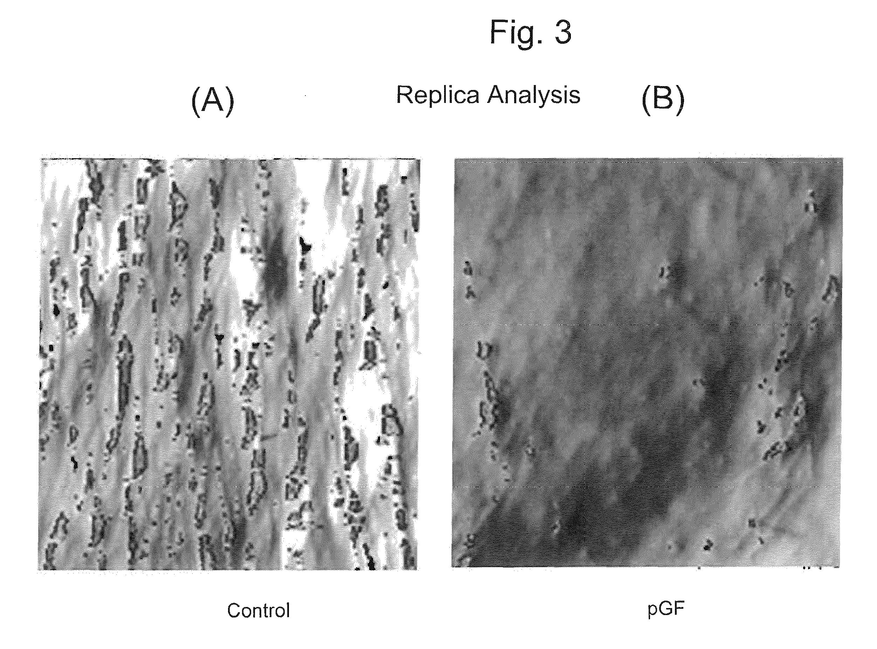

During UV exposure, fine wrinkling on the mouse skin were observed. In the treatment, it was observed that both pSHED-CM treatment group and pSHED injection group had less wrinkling than those of the PBS administration group to which PBS was solely injected (see Fig. 3 (A) and (B), in each group n=8).

Both in Figs. 3(A) and (B) showed that the wrinkling was reduced by the repetitive administration of pSHED-CM (in the figure, pSHED-CM repetitive administration group was shown as "pGF".). The pSHED injection group showed the same trend as that of pSHED-CM group.

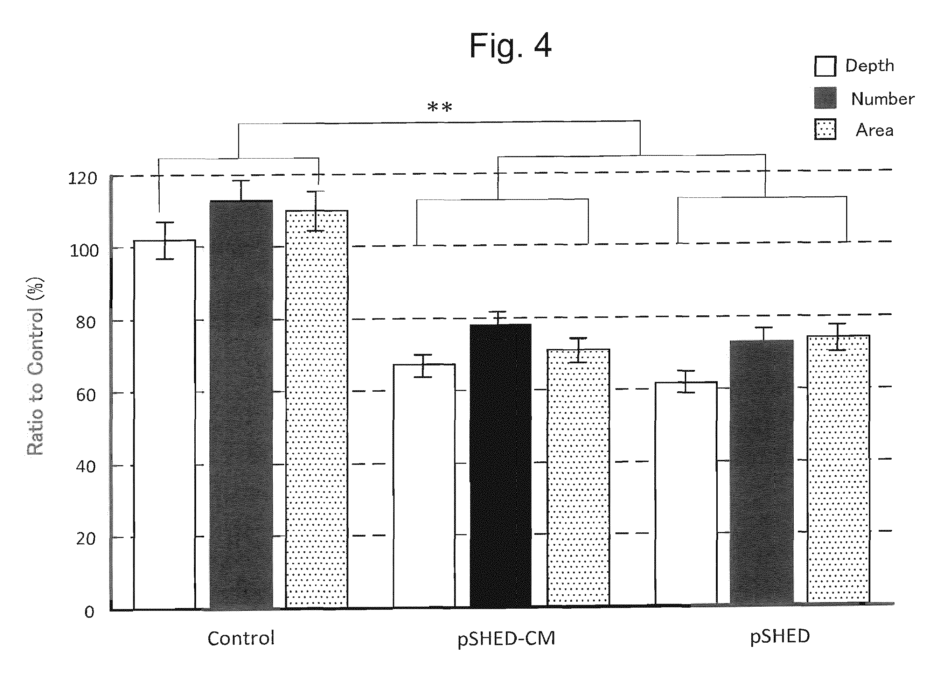

As shown in Fig. 4, the wrinkle parameters were determined by using the skin visiometer SV600. All of the wrinkle parameters were significantly reduced, when the natural level of pSHED-CM (100 %) was injected. Also, the pSHED treatment showed that higher availability than pSHED-CM treatment.

### (3) Tissue observation

Since the UVB irradiated hairless mouse showed large change in the appendage of the skin, the effect of pSHED-CM against the thickness of the dermis in the UVB irradiated hairless mouse was investigated.

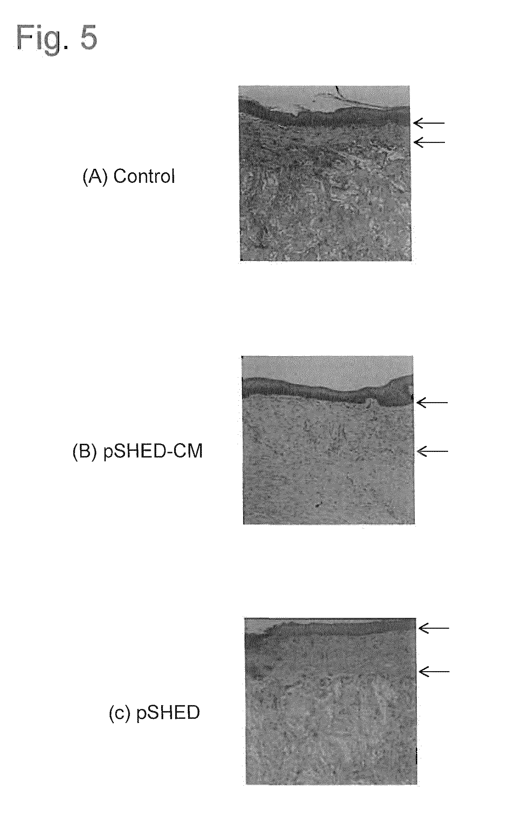

Fig. 5 shows the histological staining results of the dermis thickness of the hairless mouse skin with the hematoxylin-eosin staining (H & E staining). As shown in Figs. 5 (A) to (C), the amount of collagen fibers (the part sandwiched by the up and down arrows in the figure) significantly increased compared to the control group in the pSHED-CM treatment group (Fig. 5 (B)) and the pSHED injection group (Fig. 5 (C)).

Also, the measurement of the dermis thickness showed that the dermis thickness significantly increased both in the pSHED injection group and the pSHED-CM treatment group (Fig. 5 (B) and (C)). Furthermore, it was observed the significant increase of the bundle of collagenous fibers in both of the groups, but it was not observed in the control group (Fig. 5 (A)).

### (4) HDF growth promotion by pSHED-CM

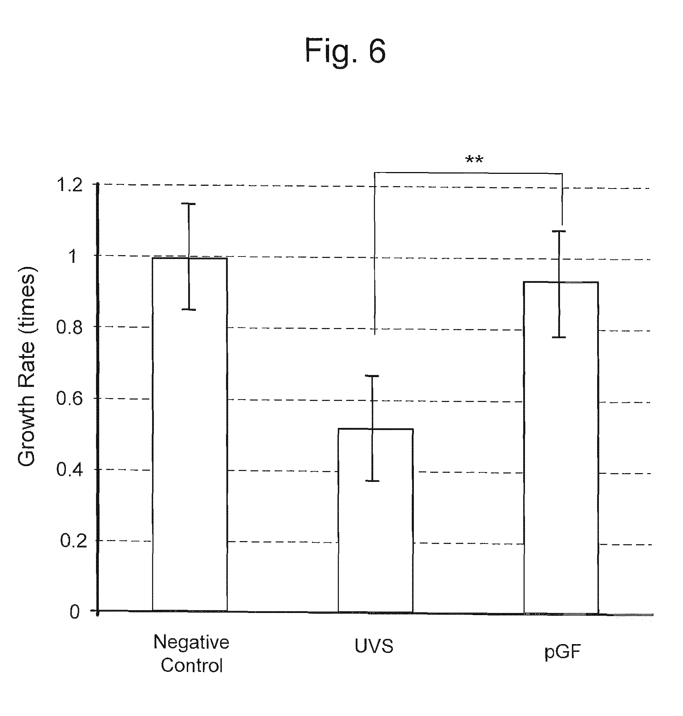

In order to study a paracrine mechanism related to wrinkle amelioration of the skin with the swine SHED, the cell growth assay was performed by using HDF which was primarily cultured with pSHED-CM under the same conditions as described above. As shown in Fig. 6, UVB irradiation significantly reduced the HDF growth (in Fig.6, it was shown as "UVS"). However, the pre-treatment by the swine SHED-CM diminished the growth declining width (In Fig. 6, it was shown as "pGF".). This means that the pSHED-CM has the protection effect to HDF.

Since the pSHED-CM comprised a variety of growth factors, it suggested that the growth promotion by the swine SHED-CM was mediated by the growth factors secreted from pSHED.

### (5) Expression of type I collagen and MMP1

In the hairless mouse treated with the swine SHED-CM, the collagen content in the dermis significantly increased. Therefore, we studied the both protein expression of the type I collagen and MMP1 in HDF after the pSHED-CM treatment. Due to the UVB irradiation, the type I collagen expression amount clearly decreased, but the expression of MMP1 increased by the induction.

Also, the expression amount of the type I collagen significantly increased compared to after the pSHED-CM pre-treatment. In contrast, the expression amount of MMP1 decreased after the pSHED-CM pre-treatment. As a result, it was shown that the increase of the collagen content in the hairless mouse dermis with the pSHED-CM treatment was caused by stimulating the collagen synthesis in the dermis fibroblast and inhibiting the collagen lysis.

### EXAMPLE 2

Production of the growth factor cocktail derived from the swine SHED/BM/ adipose stem cells and evaluation of quality thereof

### (1) Preparation of the growth factor cocktail (powder)

The stem cells of the swine SHED, swine BM or swine dental pulp were used to prepare the cocktail of the swine growth factor (pGF). By using DMEM containing 10 % FCS, the stem cells of the swine SHED, BM or dental pulp were cultured in the dish from 2 to 8 passages. When the cells in the dish became 80 % confluent, 10 mL of sup (culture medium: CM) was sampled. Nine volume of ethanol was added to the 10 mL of the sample to prepare ethanol diluted CM, which was incubated at -20 °C for 60 minutes. Then, it was transferred into the spin column of 50 mL volume and centrifuged at 4 °C for 15 minutes in 15,000 rpm to obtain precipitates. The precipitates were washed with 90 % ethanol at -20 °C, and then it was applied on another spin column and again centrifuges as described above.

The precipitates condensed as mentioned above were lyophilized to obtain a powder including the growth factors.

### (2) Growth factors in the powder

The growth factors in the powder were analyzed by using Western blotting method as described above. Detected growth factors were PDGF, VEGF, IGF, KGF, HGF, and TGF.

### (3) Quality evaluation

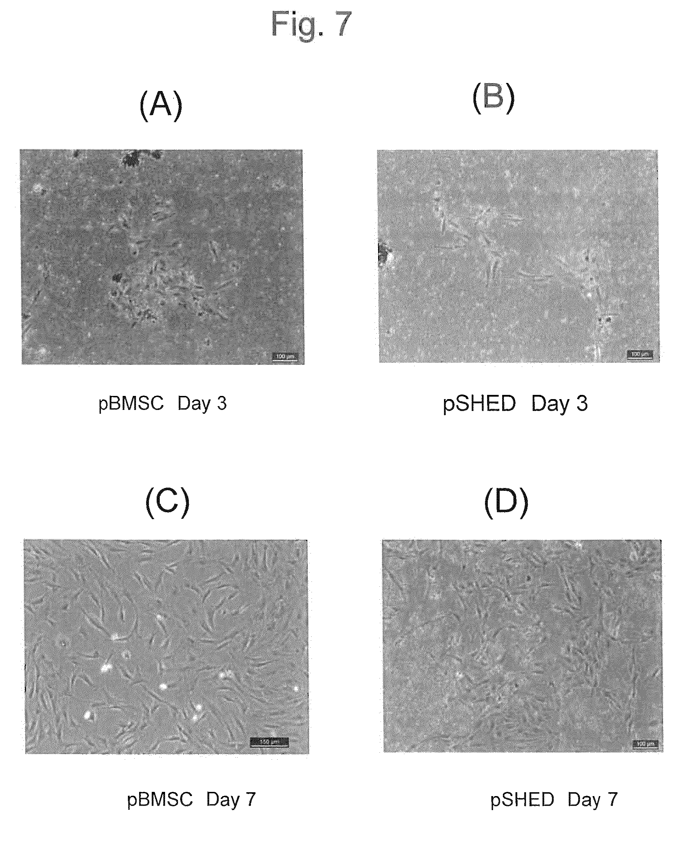

The results during the cell culture with the culture sup of the swine cultured-stem cells or the swine bone marrow stem cells are shown in Figs. 7(A) to (D). It was observed that both of the culture sup promoted the cell growth rates compared to the rate without the culture sup.

### EXAMPLE 3

Results by the culture sup of the dental pulp stem cells derived from the non-human animal against the skin

### (1) Preparation of the culture sup of the dental pulp stem cells derived from the non-human animal

The lyophilized powder was prepared by taking 1 mL of the culture sup obtained from the non-human animal (swine) in the example 2 and lyophilizing of it.

The lyophilized powder was dissolved in 30 mL of the physiological saline to be transferred into a washing bottle, and prepared the sample No. 1 for the skin to test its effect to the skin.

### (2) Test conditions for the skin

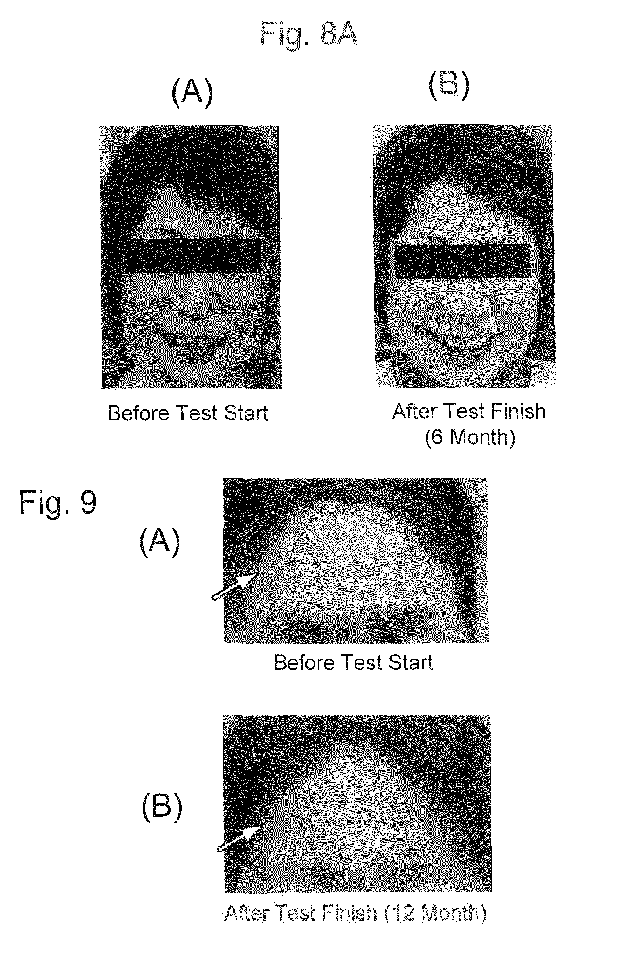

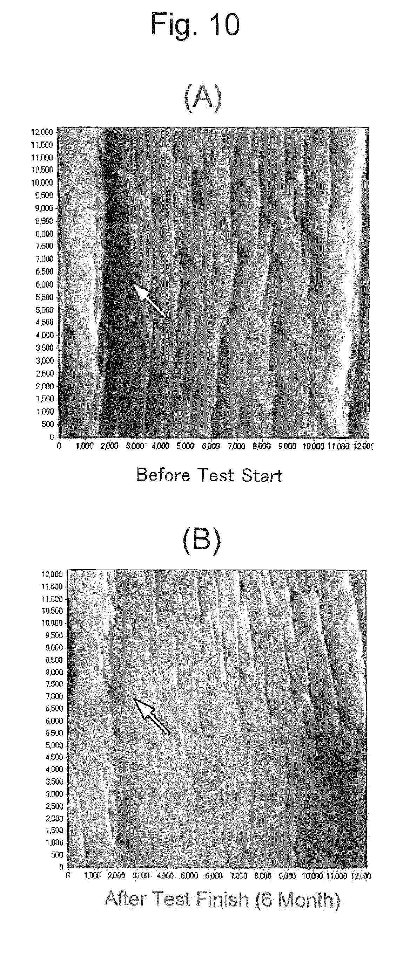

The test for the skin was carried out according to the following procedures. Firstly, the skin condition of the test subject was checked, and performed an interview how they consider their skin conditions. Next, the entire face conditions of the subjects were pictured by a camera before starting the test (see, Fig. 8 (A)), and their skin conditions were pictured by using the microscope (x 200) (see, Fig. 10 (A)).

Next, whole amount of the sample No.1 was perfused in the non-woven fabric for the face pack (Fuji Paper Chemical Company, Facemask). The non-woven fabric was placed on the face of the subject, and the iontophoresis was performed under the conditions for 20 minutes per time and 1 or 2 times/week at 0.2 to 1 mA.

During the iontophoresis, the steamer (Takara Belmont, Noage) was placed at the position, 0.3 to 0.5 m from the face for providing the steam on the entire of the face. After that, the facepack was removed from the face, and the face was treated with a cream and the like to finish. The test subjects were 7 women and their ages were 20's to 70's, average age 41.

### (3) Effects

The conditions after the test, 14 times iontophoresis, were confirmed by using the microscope as the same as that before the test start. Compared to the skin conditions before and after the tests, the skin color of the subjects became totally whiter than before the test start. Also, the spots were faded (Figs. 8 (A) and (B)), and the wrinkles were reduced (Figs. 9 (A) and (B)).

When the skin conditions were observed by using the microscope, there were clear changes to reduce the depth of the wrinkle dent (Figs. 10 (A) and (B)).

It was demonstrated that the cosmetic including the culture sup of the swine dental pulp stem cells has the effects to reduce the depth of the wrinkle and lightening the skin color.

### EXAMPLE 4

Study for growing hair and making hair thick

### (1) Preparation of the culture sup derived from the human adipose stem cells

The lyophilized powder was prepared by taking 1 mL of the culture sup obtained from the non-human adipose cells (swine) in the example 2 and lyophilizing of it.

The lyophilized powder was dissolved in 30 mL of the physiological saline to be transferred into a washing bottle, and prepared the sample No. 2 for the growing hair and making hair thick.

### (2) Test conditions for growing hair and making hair thick

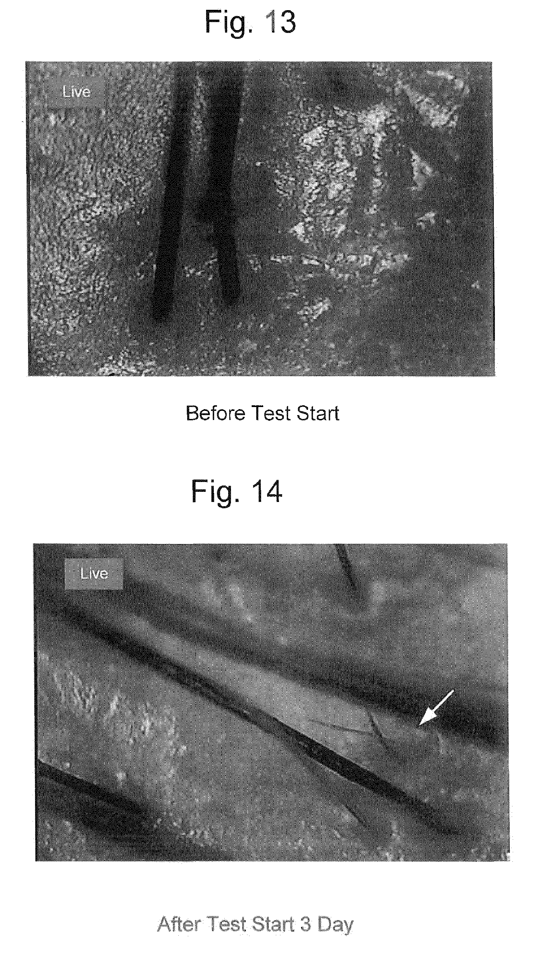

The test for the growing hair and making hair thick was carried out according to the following procedures. Firstly, the hair and the scalp conditions of the test subject was checked, and performed an interview how they consider their hair and the scalp conditions. Next, the hair conditions of the subjects were pictured by a camera before starting the test, and their scalp conditions were pictured by using the microscope (x 20 to 200). Figs. 13 to 16 were pictured with x 200.

Next, appropriate volume of cleansing gel (National total Product, Pleasure Cleansing Gel) was poured in the palm to be applied onto the scalp. Then, the scalp was massaged by hands to release pore-clogging debris or those around the pore. Subsequently, the release debris was washed out by using the commercially available shampoo (Cosmenist, Pleasure C Shampoo). When the debris amounts were much, shampoo was performed twice.

After that, the sample No. 2 prepared as described above was attached to the scalp by contacting the nozzle of the washing bottle to the scalp. Also, the sample No. 2 was applied on the hair.

The iontophoresis induction was performed under the conditions for 15 minutes per time and 1 or 2 times/week at 0.2 to 1 mA. Subsequently, the entire of the scalp and the hair were wrapped by using polyethylene film. It was further covered by using the towel like a turban to perform pack for 10 to 20 minutes. After that, the towel and the polyethylene film were removed, and then the hair was finished with towel dry or by using a dryer. The test subjects were 8 adults, including 6 men and 2 women (Ages: 30's to 50's, average 38).

After the iontophoresis as described above, the subject by themselves were applied to the sample No. 1 or 2 to washed and clean scalp (skin). They applied the sample directly on their scalp by contacting the nozzle of the container, and then massaged their scalp for a couple of minutes so as to rub therein. Also, they applied the sample on their hair.

### (3) The effects for growing the hair and making the hair thick

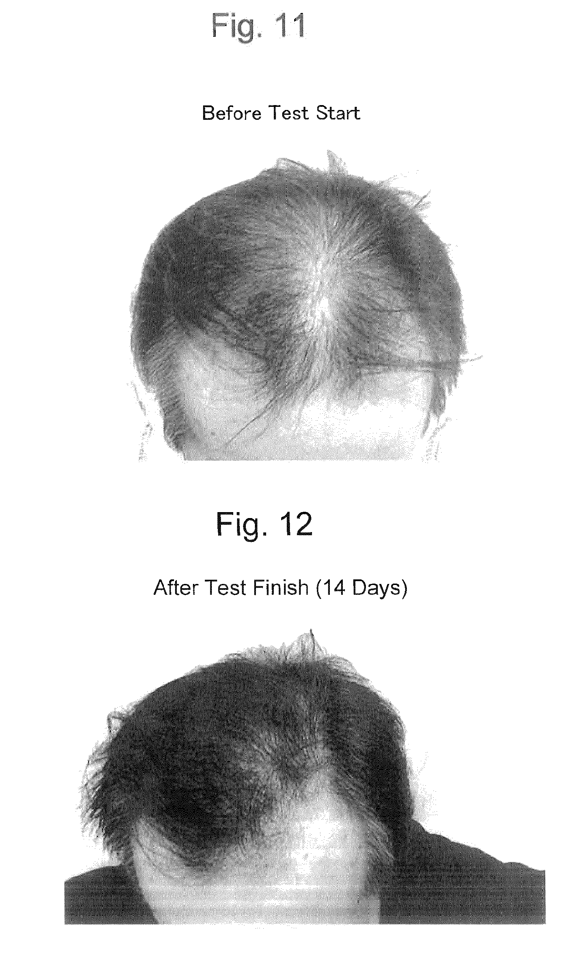

The changes of the situation of male subject before and after the test were shown in the Figs. 11 to 16.

Fig. 11 shows the hair condition at the test starting. That of the 7 days from the test start was shown in Fig. 12. Comparison of Figs, 11 and 12 clearly shows the increase of the hair in macroscopic.

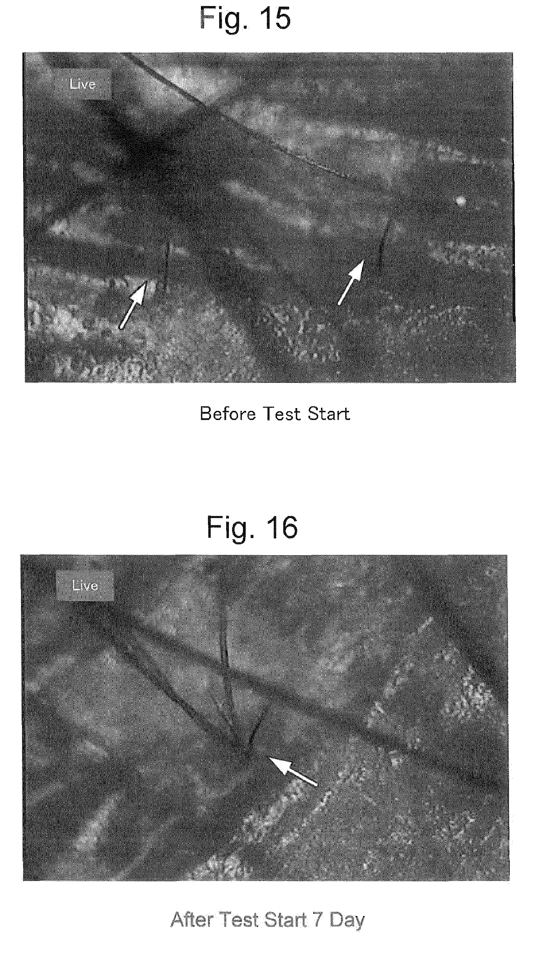

The changes after the test start were shown in Fig. 13 (before test start), Fig. 14 (after 3 days), Fig. 15 (after 5 days), and Fig. 16 (after 7 days). As the results of these observations, the hair growing has been confirmed at 3 days after the test start (see, the arrow in Fig. 14). It was confirmed that plural hairs were growing from one pore after 7 days (see the arrow in Fig. 16).

In all of the 8 subjects, the effects for the hair growing and making the hair thick were observed. Specifically, newly grown hair was black, even when the hair of the subject has partially white hair. Also, in all of the subjects, the newly grown hair was grown quickly and thick. Therefore, the volume on the top of the head increased.

The subject felt the hair growing in the earliest stage was the next day of the test start (1 day after).

Accordingly, it was confirmed that the cosmetic including the culture sup of the swine adipose stem cells has the remarkable effects for growing the hair and making the hair thick.

### Example 5

Preparations of the cosmetic comprising the growth factor derived from pSHED/pBMSC are shown in below.

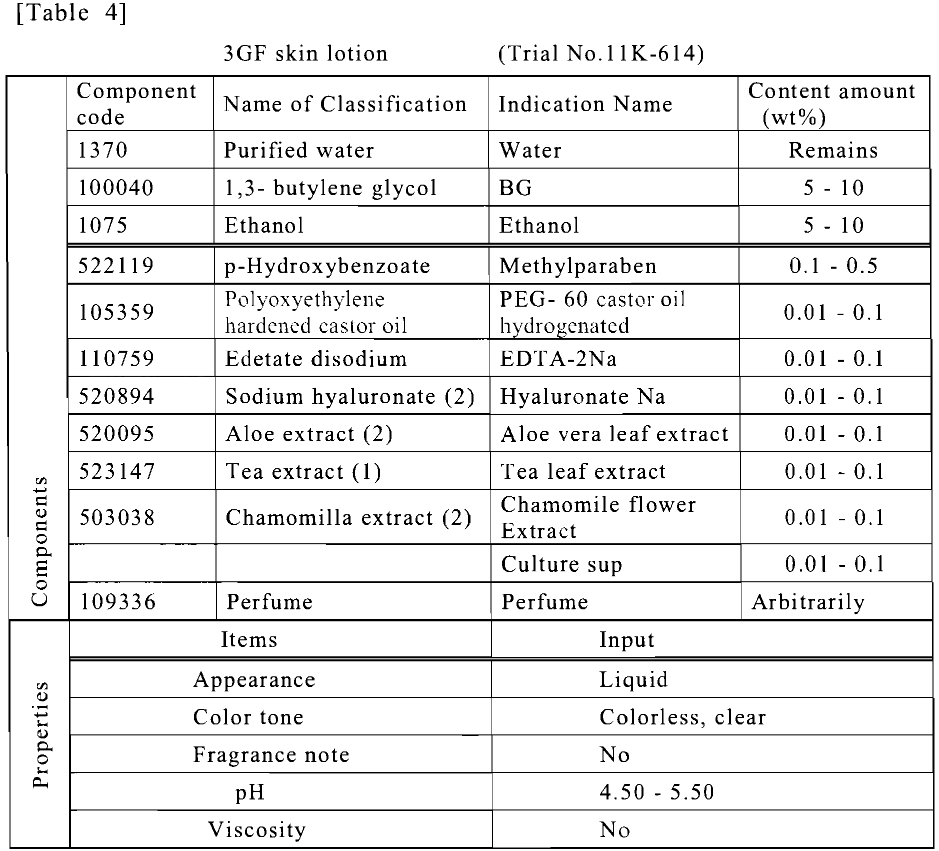

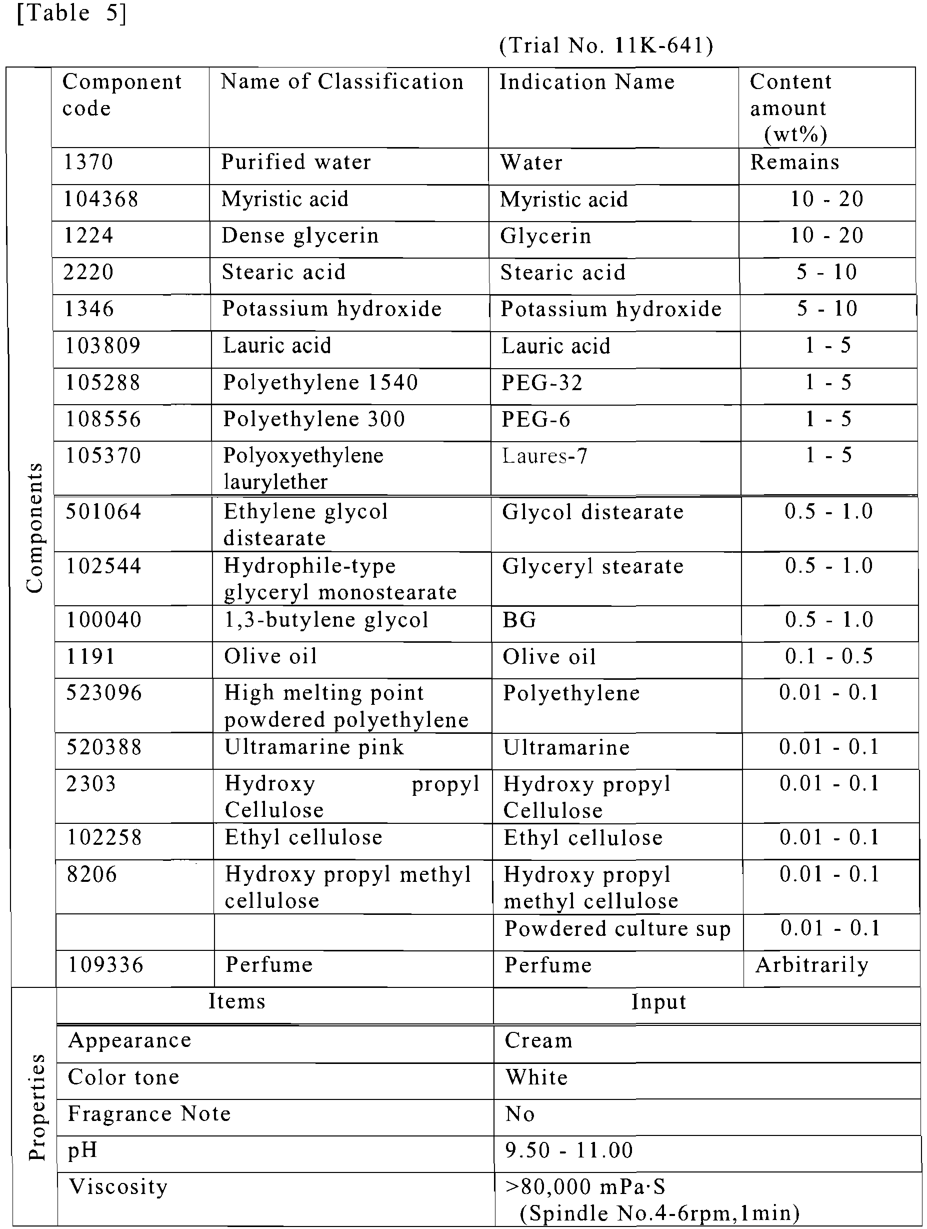

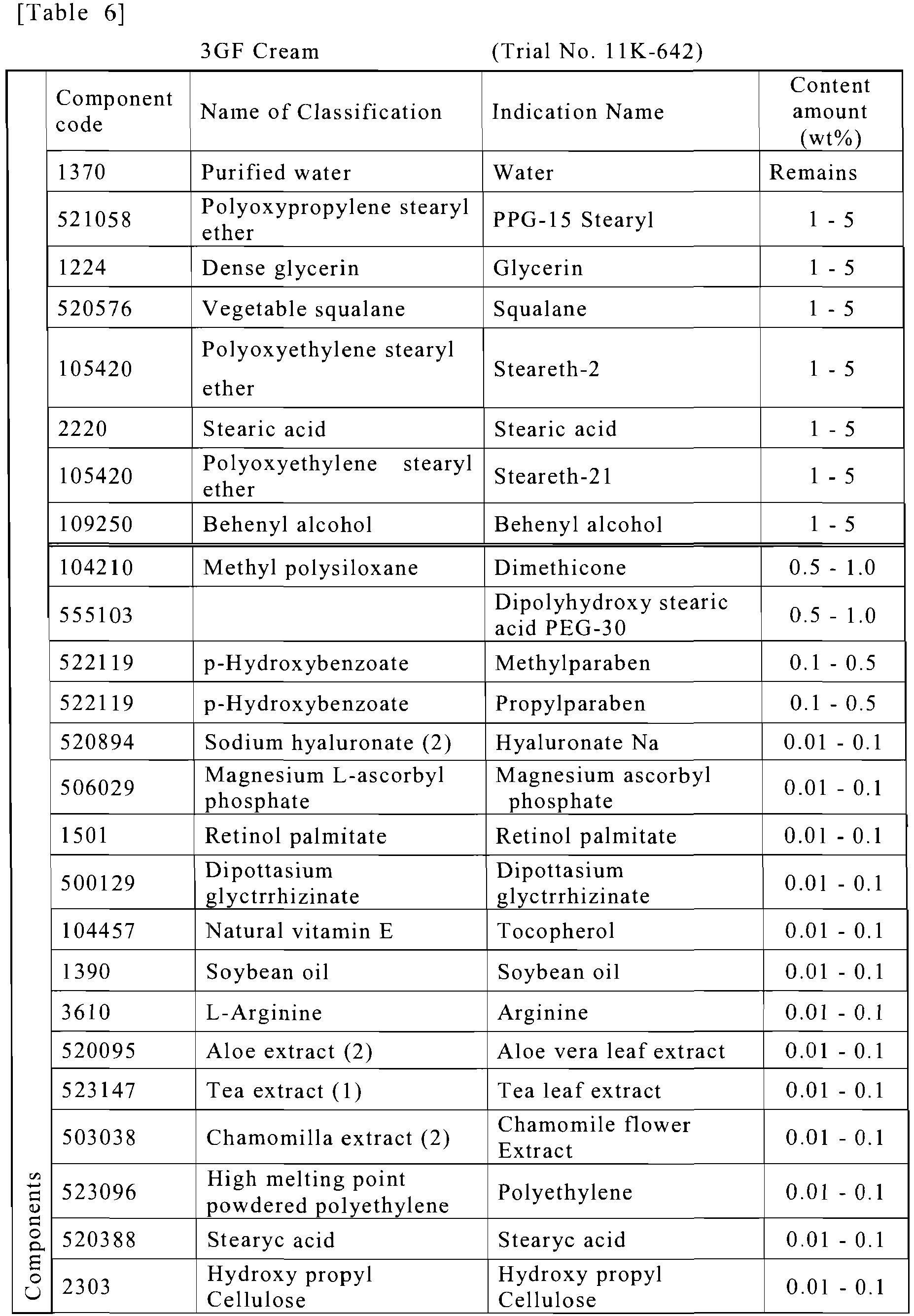

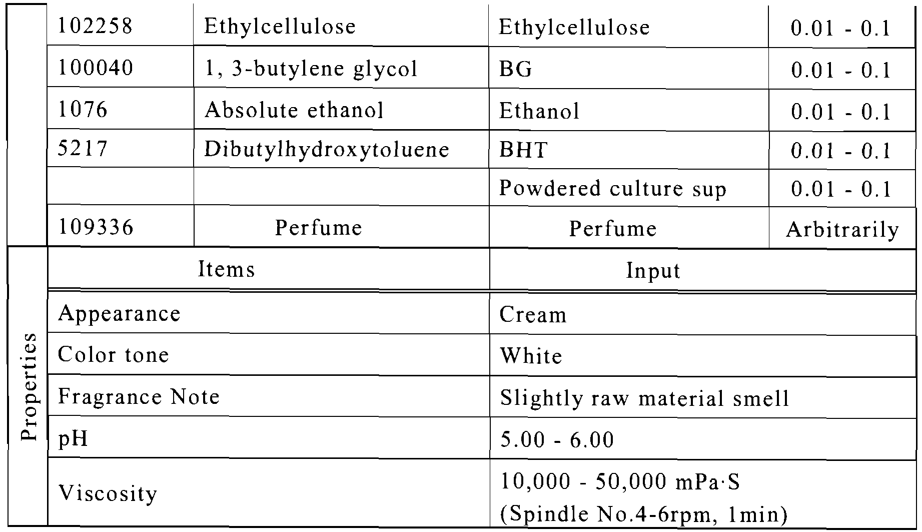

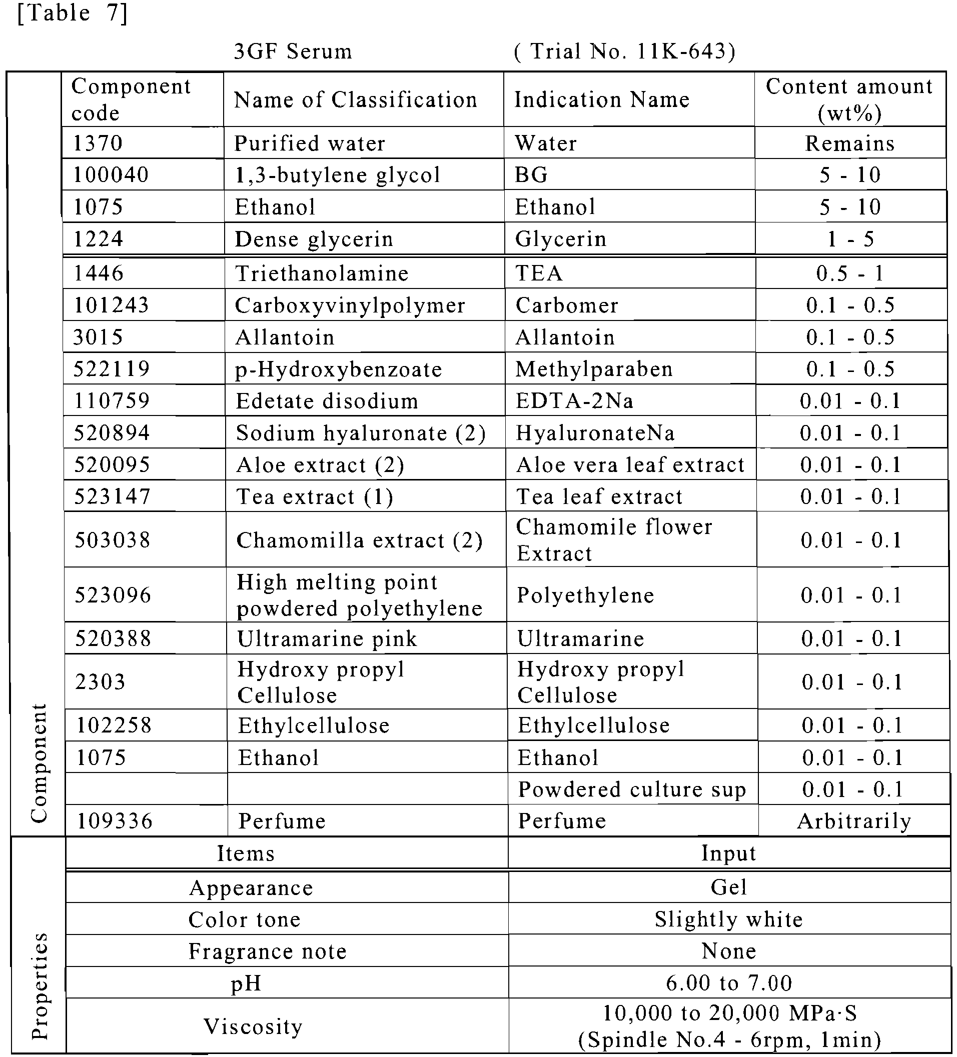

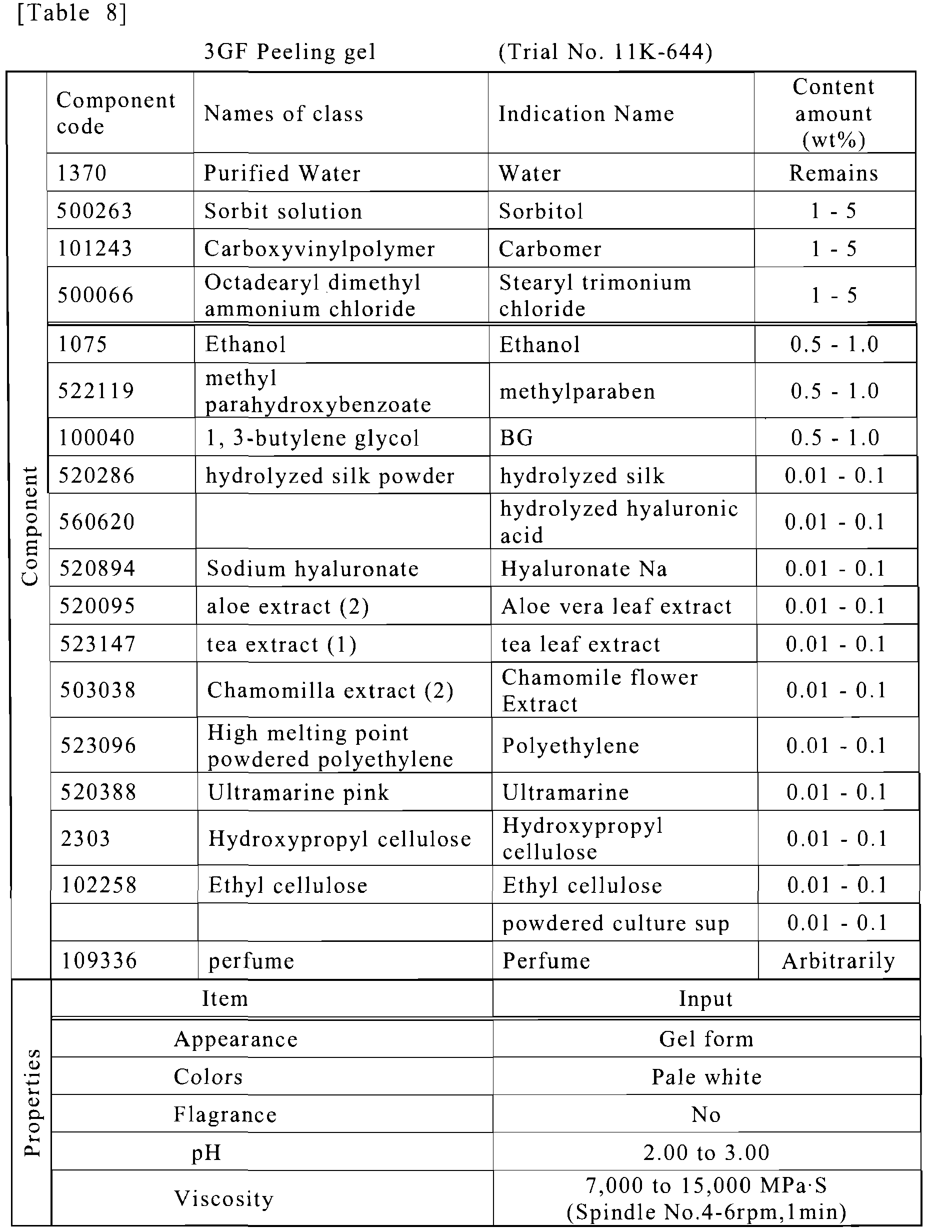

### Industrial Applicability

The present invention is useful in the cosmetic field.

## Claims

1. A cosmetic comprising any one of powdery culture sup of stem cell selected form the group consisting of a non-human mammal dental pulp stem cell, a non-human bone marrow stem cell and a non-human adipose stem cell as a main ingredient.

2. The cosmetic according to the claim 1, said non-human stem cell is obtained from any one of tissue selected from the group consisting of a swine dental pulp of an exfoliated deciduous teeth, a swine bone marrow, and a swine adipose tissue.

3. The cosmetic according to the claim 2, said powdery culture sup is prepared from a culture sup selected from the group consisting of said dental pulp of said exfoliated deciduous teeth, said bone marrow, and said adipose tissue of the swine by freeze-drying after alcohol condensation.

4. The cosmetic according to the claim 1, said powdery culture sup comprises at least one of a growth factor selected from the group consisting of platelet-derived growth factor (PDGF), vascular endothelial growth factor (VEGF), insulin-like growth factor (IGF), keratinocyte growth factor (KGF), hepatocyte growth factor (HGF), and transforming growth factor (TGF).

5. A cosmetic according to any one of the claims 1 to 4, said cosmetic form is anyone selected from the group consisting of a liquid, a cream, an ointment, a gel, an emulsion, and a cataplasm.

6. A cosmetic according to the claim 5, said cosmetic is applied for skin including scalp or hair.

7. A cosmetic stored in a container comprising
a first compartment,
a second compartment,
a bar member to form a through hole on a partition between said first and said second compartment; wherein,
said first compartment includes a solvent; said second compartment includes an active ingredient composition comprising a carrier and any one of powdery culture sup selected from the group consisting of that of a swine dental pulp exfoliated deciduous teeth, that of a swine bone marrow, and that of a swine adipose tissue; and said bar member formed said through hole in said partition to solve said active ingredient composition into said solvent immediately before to use said cosmetic.

8. The cosmetic according to the claim 6, said solvent is any one of selected from the group consisting of a liquid to which an ion is charged, saline and phosphate buffered saline.

9. The cosmetic according to any one of the claims 7 and 8, which is applied for skin including scalp and hair.

10. A an iontophoresis method for a protein comprising the steps of:
placing a sheet form of a moisture-retaining member containing said cosmetic according to any one of the claims 3 and 7 on a predetermine site through which said cosmetic is absorbed;
attaching a positively-charged electrode to predetermined site; and
attaching a negatively- charged rod-like electrode to rotate and move on said sheet from.

11. The method according to the claim 10, wherein said predetermined site is any one of selected from the group consisting of an arm, a hand, a palm, a leg, and a ham, and a bottom of foot.

12. A dermal formation promoting agent comprising a powdery culture sup of a stem cell of non-human mammal selected from the group consisting of that of a dental pulp stem cell, that of a bone marrow stem cell, and that of an adipose stem cell.

13. The dermal formation promoting agent according to the claim 12, wherein said stem cell of said non-human mammal is obtained from any one of the tissue selected from the group consisting of said dental pulp tissue of an exfoliated deciduous teeth, said bone marrow tissue, and said adipose tissue.

14. The dermal formation promoting agent according to the claim 12, said powdery culture sup is prepared from said culture sup selected from the group consisting of said dental pulp of said exfoliated deciduous teeth, said bone marrow, and said adipose tissue by freeze-drying after alcohol condensation.

15. The dermal formation promoting agent according to any one of the claims 12 to 14, said powdery culture sup comprises at least one of a growth factor selected from the group consisting of platelet-derived growth factor (PDGF), vascular endothelial growth factor (VEGF), insulin-like growth factor (IGF), keratinocyte growth factor (KGF), hepatocyte growth factor (HGF), and transforming growth factor (TGF).

16. The dermal formation promoting agent according to the claim 15, said agent has any one of the form selected from the group consisting of a liquid, a cream, an ointment, a gel, an emulsion, and a cataplasm.
